# EUROPEAN PATENT APPLICATION

(11) **EP 2 377 918 A1**
(43) Date of publication of application: **19.10.2011**
(21) Application number: 10004041.9
(22) Date of filing: 16.04.2010
(51) Int. Cl.: C12M 1/12, C12M 1/40, C12P 7/10

(54) **Process for the direct production of fermentation products from biomasses in a biofilm reactor**

(71) Applicant: ETH Zurich, 8092 Zurich (CH)
(72) Inventor: Studer, Michael Hans-Peter, 8184 Bachenbülach / ZH (CH); Brethauer Studer, Simone, 8184 Bachenbülach / ZH (CH)

(57) **Abstract**

A dense but oxygen permeable membrane (28) separates the oxygen supply compartment (30) from the fermentation compartment (31), which contains all microorganisms, a nutrient medium and the pretreated lignocellulose. The oxygen, necessary for the growth and the activity of the aerobic cellulolytic enzymes producing microorganisms is solely transported from the oxygen supply compartment (30) through the membrane (28), which leads to an oxygen gradient within the biofilm growing on the membrane (28). The oxygen rich zone of the biofilm lies on the membrane (28) whereas the biofilm further away from the membrane as well as the surrounding nutrient medium are oxygen depleted. In the aerobic biofilm the extra-cellular enzymes are produced *in situ* and are released into the nutrient medium where they hydrolyse the cellulose and hemicellulose into soluble monosugars, which are then converted to the desired fermentation product by suitable microorganisms in the anaerobic zones of the reactor (21). The process can be run in batch mode as well as in a continuous mode.

## Description

### FIELD OF THE INVENTION

The invention relates to a process for the microbial production of fermentation products from organic feedstock in a reactor according to the preamble of claim 1. The invention further relates to a device for the microbial production of fermentation products from organic feedstock in a reactor according to the preamble of claim 10.

### BACKGROUND OF THE INVENTION

Organic feedstock, in particular lignocellulosic biomasses such as wood, straw, miscanthus, switch grass or municipal solid waste are an interesting alternative to starch-or sugar containing feedstock for the microbial production of fermentation products with powerful economic and ecological benefits. Lignocellulose is a recalcitrant material composed of cellulose (40-50%), hemicellulose (25-30%) and lignin. The recalcitrance of lignocellulose makes it much more difficult than starch to enzymatically degrade to fermentable sugars. Thus, lignocellulosic materials are usually subjected to a thermochemical pretreatment step that loosens up the lignin-cellulose fiber entanglement to improve enzyme access to the cellulose. Several different pretreatment methods employing e.g. dilute sulfuric acid, steam, hot water, ammonia or lime are possible and give high sugar yields in the subsequent enzymatic hydrolysis. The acidic to neutral preteatments typically solubilize a large fraction of the hemicellulose, whereas the basic pretreatments tend to dissolve the lignin fraction. Furthermore, several lignin or sugar degradation products are released during pretreatment, such as acetic acid, HMF or furfural, which may inhibit the subsequent hydrolysis and fermentation. Typically, the liquid and the solid phase are separated after the pretreatment step, and the solids are thoroughly washed with water. If during the pretreatment step most of the hemicellulose is solubilised to xylose, a C5-sugar, or xylo-oligomers, as it is the case under neutral or acidic conditions, the liquid phase is detoxified by treatment with lime to deactivate the above mentioned fermentation inhibitors. The detoxified C5 sugar solution is then converted to the desired end product, e.g. ethanol, with the aid of special microorganisms with the ability to metabolize such sugars. The washed solids are treated with a mixture of cellulolytic enzymes and thereby hydrolyzed to mono-sugars. These enzymes are either purchased commercially, e.g. Spezyme CP , Accelerase 1500, Accelerase BG, Accelerase DUET (Genencor International, USA), Novozyme-188, Cellic CTec2, Cellic HTec2 (Novozyme, Denmark) or are produced on site, e.g. by an aerobic culture of *Trichoderma reseei* in a stirred tank reactor. In order to avoid the inhibition of the enzymes by the released sugars, microorganisms, e.g. yeast, are often added at the same time, which convert the sugars as soon as they are released to the desired fermentation product. Finally, the fermentation product from both production streams is isolated and purified by a suitable method, e.g. distillation.

It is obvious that the described process is very complex and necessitates an elaborated and costly plant, which leads to elevated costs for the desired end product. Furthermore the complex process cannot be carried out in one single reactor.

### DESCRIPTION OF THE INVENTION

It is therefore the object of the invention to create a process and a device for carrying out the process, which is less complex and more cost-efficient by integrating several process steps.

These objects are achieved by the features of the characterizing part of the claims 1 and 10. Further preferred embodiments are evident from the dependent patent claims.

The reactor contains an oxygen permeable membrane, which separates an oxygen supply compartment or chamber from a fermentation compartment or chamber. The oxygen supply compartment is designed for accommodating and circulating a fluid, which contains oxygen (molecular oxygen). The oxygen can be supplied to the oxygen supply compartment in a gaseous phase. In this case it is also possible that a mixture of gas, containing at least oxygen, e.g. air, is supplied to the oxygen supply compartment and hence to the surface of the membrane. However, it is also possible that the oxygen, which is supplied to the oxygen supply compartment, is dissolved in a liquid. The liquid can be water-based. It is also possible that the liquid is a silicone oil or any other solvent, which has excellent gas absorption properties. In the first case a gas (gaseous oxygen or a gas mixture containing at least gaseous oxygen) is circulated in the oxygen supply compartment and in the latter case a liquid is circulated in the oxygen supply compartment.

The oxygen supply to the fermentation compartment, i.e. the amount of oxygen transported through the membrane within a time period can be controlled by means of at least one of the following parameters:
- oxygen pressure or oxygen partial pressure if the oxygen is supplied as a gas to the oxygen supply compartment or the amount of oxygen dissolved in the liquid if the oxygen is dissolved in a liquid and supplied with the liquid to the oxygen supply compartment,
- composition (e.g. density, material, porosity) and the thickness of the membrane.

The fermentation compartment is designed for accommodating a base material mixture containing organic feedstock, particularly a water-based solution or a water-based suspension containing organic feedstock, optionally additional nutrients, e.g. complex nitrogen source (corn steep liquor), phosphate, sulphate, trace elements or vitamins and surfactants.

The membrane is a layer of material, which serves as a selective barrier between the oxygen supply compartment and the fermentation compartment and remains impermeable particularly to the liquid, to the organic feedstock and to the microorganisms. On the other hand the membrane allows the passage of at least oxygen and preferably also of the fermentation products such as alcohols. The membrane can be of various thicknesses and it can be a flexible or rigid layer.

The reactor contains means for continuously or discontinuously supplying oxygen into the oxygen supply compartment. The means can be designed to circulate an oxygen-containing liquid or an oxygen-containing gas in the oxygen supply compartment. Furthermore the reactor contains means for continuously or discontinuously supplying organic feedstock into the fermentation compartment.

The organic feedstock is preferably suspended in a water-based medium. In this case the aqueous suspension is supplied into the fermentation compartment e.g. by means of a pump. However it is also possible that a highly viscous mass with a high content of solid organic feedstock is supplied to the fermentation compartment instead of an aqueous suspension of low viscosity. For example a mixture of water and organic feedstock with only 20% of solid organic matter has almost the properties of a solid body and can e.g. not be pumped into the fermentation compartment like a liquid. However, as soon as a part of the organic feedstock (e.g. cellulose, hemicellulose) is broken down by the enzymatic process, the highly viscous mass fluidifies to a suspension of low viscosity.

On the surface of the membrane facing the fermentation compartment a biofilm is located, which contains at least one species of aerobic microorganisms, i.e. the membrane is partly or completely covered with the biofilm. In comparison to known biological processes where the microorganisms are suspended in a water-based solution and therefore fully mobile, the microorganisms of the biofilm according to the present invention are immobilized on the membrane and do therefore not flow with the aqueous suspension.

Means, as e.g. a support structure like a supporting mesh, can be provided on the membrane, next to the membrane or can be integrated into the membrane. The membrane can be part of a composite structure comprising the membrane itself and a supporting structure. The support structure can have one or both of the following functions:
- securing the biofilm on the membrane, so that the biofilm can not be eroded by the flow of the liquid or suspension within the fermentation compartment;
- supporting the membrane, particularly if the membrane is thin and flexible and therefore not self-supporting.

If the support structure serves as a securing means for the biofilm then the support structure is preferably provided on the surface of the membrane or next to the surface of the membrane accommodating the biofilm. The support structure is preferably affixed to the membrane.

If the support structure serves as a support for the membrane itself then the support structure can either be affixed to the membrane or be embedded in the membrane. The membrane and support structure can e.g. be a composite structure, where the support structure is e.g. molded in the membrane. Having a support structure which carries the membrane, the use of a thin membrane with high gas permeability is possible.

The support structure may act as a support for the membrane and as a securing means for the biofilm as well. However, separate support structures may also be provided: a first support structure which acts as a support for the membrane and a second support structure, which acts as a securing means for the biofilm.

Oxygen is transported from the oxygen supply compartment to the fermentation compartment through the membrane and an oxygen rich zone is formed in the fermentation compartment adjacent to the membrane, i.e. directly on the membrane. The strain of aerobic microorganism of the biofilm is located adjacent to the membrane, i.e. directly on the membrane, in the aerobic zone. An oxygen gradient with decreasing oxygen content with increasing distance from the surface of the membrane is formed within the biofilm.

The fermentation compartment further contains at least a strain of anaerobic microorganism for the fermentation of fermentable sugar to fermentation products. The anaerobic microorganism can be part of the biofilm or can be located in the base material mixture. I.e. the anaerobic microorganism can be suspended in a water-based suspension containing organic feedstock. The suspended anaerobic microorganism can be located on organic material suspended in the water-based suspension.

The strain of aerobic microorganism is preferably part of a consortium of at least two species or strains of microorganisms. The consortium contains at least a strain of aerobic microorganism, which is located in the aerobic zone of the fermentation compartment and a strain of anaerobic microorganisms in an anaerobic zone of the fermentation compartment. Both aerobic and anaerobic microorganisms are preferably located in the biofilm. The aerobic microorganisms, however, are located in an aerobic zone of the biofilm, whereas the anaerobic microorganisms are located in an anaerobic zone of the biofilm. Anaerobic microorganisms can also be located in the biofilm and in the base material mixture as well, e.g. in the suspension, containing organic feedstock.

In the reactor the following steps take place:
a. production of enzymes for the enzymatic degradation of organic feedstock to fermentable sugar by the strain of aerobic microorganism in the aerobic zone;
b. enzymatic degradation of organic feedstock to fermentable sugars;
c. fermentation of the fermentable sugars to fermentation products by the strain of anaerobic microorganism in the anaerobic zone.

If the organic feedstock is cellulose or hemicellulose, as e.g. contained in lignocellulose, then aerobic microorganisms are used, which produce cellulases (a class of enzymes that catalyse the hydrolysis of cellulose). If the organic feedstock is starch then aerobic microorganisms are used, which produce amylases (a class of enzymes that catalyse the hydrolysis of starch). In all cases an enzymatic hydrolysis of the cellulose, the lignocellulose, the hemicellulose or of starch to fermentable sugars takes place.

In a preferred embodiment the method further comprises the step (d) of isolating the fermentation product from the fermentation compartment. The fermentation products are preferably isolated from the fermentation compartment by transportation of said products through the membrane into the oxygen supply compartment. Once fermentation products are in the oxygen supply compartment they are subsequently removed from the oxygen supply compartment and separated. The fluid (e.g. a liquid or a gas), which circulates within the oxygen supply compartment, and which contains the oxygen may act as a carrier medium for the fermentation product. In this case the fermentation products are removed from the oxygen supply compartment by removing the fluid. The fermentation products, which passes through the membrane can e.g. enter the oxygen supply compartment in a gaseous phase and can be removed from the oxygen supply compartment with the gaseous flow. If the oxygen supplied to the oxygen supply compartment is dissolved in a liquid, which is supplied to the oxygen supply compartment, then the fermentation products, which pass the membrane and enter the oxygen supply compartment, can get dissolved in the liquid. The fermentation products can be removed from the oxygen supply compartment with the flow of the liquid.

The separation of the fermentation products can take place by means of e.g. a distillation, a condensation, an adsorption or an extraction process.

In this context the biofilm reactor preferably comprises means for removing the fermentation products from the oxygen supply compartment and for separating the fermentation products from the gaseous or liquid phase.

The process steps do not have to run in a sequential order a. to d. The process steps can run in an overlapping manner or contemporaneous.

The transportation of oxygen and/or the fermentation products through the membrane is preferably a solution diffusion process. In this case a dense membrane is used. However, it is also possible that the membrane contains pores and the oxygen and/or the fermentation products are passing the membrane through the pores. It is also possible that the properties of the membrane are such that the transport of the substances takes place through pores and through a solution diffusion process. Furthermore it is also possible that the transportation of oxygen and the fermentation products occurs through two different types of membranes.

In a preferred embodiment of the invention the biofilm contains a consortium of at least two species of microorganisms, wherein at least a first of these strains of microorganism is an aerobic strain located adjacent to the surface of the membrane in the aerobic zone of the fermentation compartment, and wherein at least a second of these strains of microorganisms is an anaerobic microorganism located in the biofilm on the membrane in an oxygen depleted, preferably anaerobic, zone of the fermentation compartment neighboring the strain of aerobic microorganism. In this case also the strain of anaerobic microorganism in the biofilm is immobilized. However, a combination of anaerobic microorganism immobilized in the biofilm and suspended in the base material mixture is also possible.

The microorganisms in the biofilm are preferably arranged in a layer structure on the membrane. If the biofilm contains a consortium of microorganisms, e.g. at least one aerobic and anaerobic strain of mircroorganism, the biofilm can have a multi-layer structure where the different species of microorganisms build single layers. The adjoining layers of different species of microorganisms can define visually clearly defined layer boundries. It is also possible that the different species of microorganisms of two layers are grown together and interwined in the transition zone, so that there is no clearly defined layer boundry visible.

The membrane, which separates the oxygen supply compartment from the fermentation compartment is preferably dense but oxygen permeable. The dense membrane is preferably made of or contains silicone, preferably polydimethylsiloxane. Further the membrane can contain or consist of fluorocarbon compounds (e.g. polytetrafluor-ethylene), hydrocarbon compounds (e.g. polyethylene, polypropylene), polysulphone or polyalkylsulphone.

The thickness of the membrane is preferably 1 micrometer or more, particularly 50 micrometer or more. Further, the thickness can be 2000 micrometer or less, particularly 1000 micrometer or less. The membrane can have a tubular shape. However, the membrane can also be flat. According to a first alternative, the oxygen supply compartment lies outside the tubular membrane, i.e. on at least a part of its outer circumference, and the fermentation compartment lies within the tubular membrane, i.e. within the tube-like space surrounded by the membrane. According to a second alternative, the oxygen supply compartment lies within the tubular membrane, i.e. within the tube-like space surrounded by the membrane, and the fermentation compartment lies outside the tubular membrane, i.e. on at least a part of its outer circumference. Depending on the embodiment, the biofilm lies either on the inner surface of the tubular membrane facing the tubular space, or on the outer surface of the tubular membrane facing the space surrounding the circumferential surface of the membrane.

If the membrane is tubular-shaped, the supply and discharge of the medium into the tubular-shaped compartment preferably occurs through the front ends of the tubular membrane. The medium is axially transported through the tubular membrane. The compartment surrounding the tubular membrane can be ring shaped. It is also possible that several tubular membranes are placed in a reactor chamber which either forms the oxygen supply or the fermentation compartment.

The base material mixture containing organic feedstock, in particular the suspension containing lignocellulose, and optionally additional nutrients, is brought in contact with the biofilm. The aerobic microorganisms within the biofilm produce enzymes, which break down, particularly hydrolyse, the organic feedstock, particularly the lignocellulose, into fermentable products, particularly fermentable sugars.

Fermentable sugars can be monosaccharides (mono-sugars, as e.g. xylose, glucose, mannose, galactose, arabinose), disaccharides (e.g. cellobiose, xylobiose, sucrose), trisaccharides (cellotriose, xylotriose) resp. oligosaccharides or even polysaccharides. Independent of the type of fermentable sugar it is important that the fermentable sugar:
a.) is fermentable into fermentation products, and
b.) is water-soluble, so that the sugar can be fermented in a water-based solution.

The anaerobic microorganisms in the biofilm and/or in the suspension ferment the fermentable sugar into fermentation products. The fermentation products are preferably organic compounds and can comprise substances from substance groups, such as e.g. ketones (acetone, diethyl ketone methyl-ethyl-ketone,), esters (methyl-, ethyl- or propyl- long-chain alkanoates), alkanes (e.g. methane ethane, propane, butane, long-chain n-alkanes), carboxylic acids (acetic, propionic, butyric, lactic acid) and preferably alcohols (e.g. ethanol, methanol, propanol, butanol). The fermentation products can either be the source material for the production of the desired end product, e.g. a bioful, or they are already the desired end product.

The supply of oxygen through the membrane is preferably controlled in such a way, that the oxygen content within the biofilm or within a zone in the liquid phase located next to the membrane and containing the biofilm decreases from the surface of the membrane with increasing distance from this surface to a level at which anaerobic condition are established, preferably decreases to approximately zero, so that the conditions in the liquid phase containing lignocellulose in the fermentation compartment beyond this zone are anaerobic.

The growth of the biofilm on the membrane can be initiated by inoculating the membrane with at least a strain of aerobic microorganism and by incubating the microorganism. Environmental conditions are applied, which allows the aerobic microorganism to grow on the membrane and to secrete enzymes, which e.g. hydrolyze the polymeric sugars contained in the lignocellulose to fermentable sugars, which are then converted by at least one other, preferentially anaerobic strain of microorganism to the desired fermentation product.

The fermentation compartment preferably contains all the microorganisms, a nutrient medium and the organic feedstock, e.g. lignocellulose. The nutrients medium and/or the organic feedstock are either dissolved or suspended in a liquid, hereinafter simply called liquid mixture. Hence, the base material mixture can be a liquid mixture. The liquid is preferably water. The oxygen, necessary for the growth and the activity of the aerobic stain of microorganism, which produces enzymes, is transported from the oxygen supply compartment through the membrane, which leads to an oxygen gradient within the biofilm growing on the membrane. The oxygen rich zone of the biofilm lies adjacent to the membrane whereas the surrounding base material mixture and preferably also the region of the biofilm, which is further away from the membrane are oxygen depleted and preferably form an anaerobic zone.

In the aerobic region of the biofilm preferably extra-cellular enzymes are produced *in situ* and are released into the base material mixture, particularly liquid mixture, where they degrade, particularly hydrolyze, organic feedstock, particularly cellulose and/or hemicellulose, into fermentable products, particularly soluble sugars, which are then transformed to the desired fermentation product by at least one strain of suitable anaerobic microorganism in the anaerobic zone of the reactor. However, it is also possible that in the aerobic region of the biofilm aerobic microorganisms produce cell-bound enzymes. In this case, in order to degrade the organic feedstock to fermentable products, either the cells containing the enzymes are released into the base material mixture, particularly into the liquid mixture, or organic feedstock from the base material mixture is temporarily incorporated in the biofilm. It is also possible that both of the two aforementioned processes take place, particularly beside the release of extracellular enzymes.

The process according to the invention can be run in batch mode as well as in a continuous mode. The reaction temperature of the process can lie above 20 and below 100° C depending on the microorganisms, which are used.

The organic feedstock can contain or consist of at least one of:
- one or several polysaccharides (e. g. cellulose, hemicellulose or starch)
- lignocellulose

The organic feedstock is preferably produced from biomass. The organic feedstock preferably contains or consists of lignocellulose. The biomass containing lignocellulose can be corn stover, straw from the cultivars e.g. wheat, barley, sorghum, rice or rye, wood, e.g. from the cultivars spruce, fir or beech, aspen, poplar or maple and especially forestry waste such as crowns and branches, further biomasses such as miscanthus, switch grass, bagasse or sugarcane leaves or the organic fraction of municipal solid waste. However it is also possible that the organic feedstock is not directly produced from biomass but from domestic or industrial waste of processed products which contains organic material, as e.g. paper or cardboard.

The aerobic strain of microorganism for producing (ligno)cellulolytic enzymes is typically a fungi, such as *Trichoderma reesei, Aspergillus niger* or *Penicillium brasilianum.* Further strains can be:

| | | | |
|---|---|---|---|
| • | *Cellulomonas uda* | • | *Microbacterium barkeri* |
| • | *Chaetomium globosum* | • | *Bretanomyces clausenii* (Synonym: *Dekkera anomala)* |
| • | *Myceliophthora thermophila* (Synonym: *Sporotrichum thermophile)* | | |
| | | • | *Thermoascus aurantiacus* |
| • | *Myrothecium verrucaria* | • | *Gloeophyllum trabeum* |
| • | *Phanerochaete chrysosporium* | • | *Lysobacter enzymogenes* subsp. *enzymogenes* |
| • | *Sporotrichum pulverulentum* | | |
| • | *Thermoascus aurantiacus* | • | *Paenibacillus glucanolyticus* |
| • | *Trichoderma longibrachiatum,* | • | *Myceliophthora thermophila* |
| | (Synonym: *T. viride)* | • | *Fusarium oxysporum* f. sp. *vasinfectum* |
| • | *Alternaria solani* | | |
| • | *Rhizopus oryzae* | • | *Pichia canadensis* |
| • | *Aspergillus japonicus* | • | *Rhizopus oryzae* |

It is possible to combine several enzyme producing aerobic strains of microorganism or to apply only one specific strain.

The aerobic strain of microorganism for producing amylases can be:

| | | | |
|---|---|---|---|
| • | *Aspergillus foetidus* | • | *Thermoanaerobacter ethanolicus* |
| • | *Bacillus amyloliquefaciens* | • | *Bacillus halodurans* |
| • | *Bacillus licheniformis* | • | *Bacillus pseudofirmus* |
| • | *Endomyces fibuliger* | • | *Nesterenkonia halobia* |
| • | *Paenibacillus macerans* | • | *Alicyclobacillus acidocaldarius* subsp. *acidocaldarius* |
| • | *Paenibacillus polymyxa* | | |
| • | *Rhizomucor miehei* | • | *Geobacillus stearothermophilus* |
| • | *Rhizomucor pusillus* | • | *Thermoanaerobacterium thermosaccharolyticum* |
| • | *Thermoanaerobacter acetoethylicus* | | |
| • | *Thermoanaerobacter brockii* subsp. *finnii* | • | *Thermoanaerobacterium thermosulfurigenes* |

It is possible to combine several enzyme producing aerobic strains of microorganism or to apply only one specific strain.

The anaerobic strain of microorganism for fermenting the released soluble sugar can be e.g. *Saccharomyces cerevisiae, Zymomonas mobilis, Pichia stipitis, Escherichia coli* KO11, or *Klebsiella oxytoca.* Further anaerobic strain of microorganism for fermenting the released soluble sugar can be *Clostridium acetobutylicum* (production of acetone, butanol or ethanol) or genetically modified *E.coli* (production of butanol, esters or long-chain n-alkanes). Further strains can be:

| **Ethanol:** | | **n-Butanol:** | |
|---|---|---|---|
| • | *Clostridium beijerinckii* | • | *Clostridium acetobutylicum* |
| • | *Clostridium saccharobutylicum* | • | *Clostridium beijerinckii* |
| • | *Clostridium thermocellum* | • | *Clostridium saccharobutylicum* |
| • | *Thermoanaerobacter brockii* subsp. *brockii* | • | *Clostridium saccharoperbutylacetonicum* |
| • | *Thermoanaerobacter brockii* subsp. *finnii* | • | *Clostridium tetanomorphum* |
| • | *Candida shehatae* | | |
| • | *Thermoanaerobacter ethanolicus* | | |
| • | *Thermoanaerobacter thermohydrosulfuricus* | | |
| • | *Pachysolen tannophilus* | | |
| • | *Kluveromyces marxianus* | | |
| • | *Mucor indicus* | | |
| • | *Fusarium oxysporium* | | |

It is possible to combine several anaerobic strains of microorganism for the fermentation process or to apply only one specific strain.

Even though present invention is preferably designed to enzymatically degrade cellulose-, hemicellulose- and/or starch-containing feedstock to fermentable sugars and to ferment the fermentable sugar to organic substances, such as alcohol, it is possible that the present invention can be applied to a similar process which is directed to the enzymatical delignification of lignocellulose to assist the hydrolysis of polysaccharides to e.g. sugars, and to ferment the fermentable products to one of the above mentioned fermentation product. In this case the aerobic microorganisms are designed to produce ligninase or more generally lignin-modifying enzymes (LME), an enzyme which is capable of catalysing the degradation of lignin. The degradation of lignin is oxidative.

Thus, the aerobic process according to the invention can be designed to carry out the enzymatical degradation of one or several polysaccharides, such as cellulose, hemicellulose or starch. Additionally the enzymatical degradation of lignin, e.g. from lignocellulose can also be provided.

In this case one or a combination of two or three groups of strains of aerobic microorganism can be applied on the surface of the membrane to form a biofilm:
- a group of strains with at least one strain of aerobic microorganism for the production of cellulases,
- a group of strains with at least one strain of aerobic microorganism for the production of amylases, and
- a group of strains with at least one strain of aerobic microorganism for the production of lignin-modifying enzymes (ligninases)

A group of strains can contain only one or several strains of aerobic microorganism.

The process according to the invention is characterized by a higher resistance of the microorganisms growing in a biofilm against toxic substances, which are e.g., produced or released during a possible pretreatment of the biomass, or which can also be the desired final product. Thus, the usually necessary washing and detoxifying steps can be omitted or reduced and the process can be run with high substrate and product concentrations, which in turn is advantageous for the subsequent product recovery and purification.

Furthermore, a higher productivity through the high cell density due to the natural immobilisation as well as a lesser substrate consumption for undesired cell growth is achieved in the described reactor system. Moreover several process steps are integrated, which results in a technically less elaborate and less expensive process.

It is emphasised that features of the method claims can also be combined with features of the device claims and vice versa.

### BRIEF DESCRIPTION OF THE DRAWINGS

The subject matter of the invention will be explained in more detail in the following text with reference to preferred exemplary embodiments which are illustrated in the attached drawings, in which:
- Figure 1: shows sugar concentrations after 97 hours during hydrolysis of Avicel with enzymes produced in a membrane reactor;
- Figure 2: shows the time course of the cellulase acitivity during the start up phase on Mandels medium;
- Figure 3: shows the production of ethanol from cellulose by the combined action of fungi and yeast;
- Figure 4: shows the production of ethanol from cellulose by the combined action of RutC30 and yeast at different liquid volumes;
- Figure 5: shows the modeling of the diffusional ethanol loss and the remaining ethanol concentration in the reactor;
- Figure 6a: shows a first exemplary embodiment of a biofilm reactor according to the invention;
- Figure 6b: shows an expanded view of a section A of the membrane bearing the biofilm according to Fig. 1 a;
- Figure 7a: shows a second exemplary embodiment of a biofilm reactor according to the invention;
- Figure 7b: shows an expanded view of a section B of the membrane bearing the biofilm according to Fig. 2a;
- Figure 8: shows schematically a possible distribution of oxygen in the gas phase in the membrane and in the biofilm on the membrane.

### WORKING EXAMPLES

### General experimental conditions

First experiments were carried out in small membrane bioreactors adapted from commercially available reusable polysulfone filter units (Catalog number 300-4000, Nalgene, Rochester, NY, USA). The membrane was installed vertically in order to prevent settling of fungal biomass and solid particles onto the membrane. The reactor contents was magnetically stirred. The reaction volume was 320 mL. Furthermore the reactor was modified to reduce the reaction volume to 30 mL.

The first membrane tested was a dense, polydimethysiloxane (PDMS) membrane with a total thickness of only 50 µm (micrometer) (OPV-2551s-30n, CM-CELFA, Schwyz, Switzerland). The membrane area was 12.6 cm².

The following strains were tested for enzyme production: *A. niger* ATTC 10864, *T*. *reesei* wild type, T. *reesei* Rut C30, *Penicillium brasilianum* IBT 20888 and *Sporotrichum thermophile.* Strains were maintained on potato dextrose agar plates stored at 4 °C. For the production of inoculum, 10 mL sterile water was added to one well sporulating plate and spores were scraped off with a Drigalsky spatula. Reactors were inoculated with 1 % of this spore suspension. Mandels medium used for enzyme production had the following basic composition for cellulose concentrations of 10 g/L or lower.

| **Component** | **Concentration [g/L]** |
|---|---|
| Cellulose | see experiments |
| KH₂PO₄ | 2 |
| (NH₄)₂SO₄ | 1.4 |
| urea | 0.3 |
| peptone | 0.75 |
| yeast extract | 0.25 |
| Trace element stock | 1 |
| MgSO4 · 7H₂O | 0.3 |
| CaCl₂ · 6H₂O | 0.4 |

For higher amounts of cellulose the amounts of all other medium components were increased accordingly. Pure cellulose (Avicel PH-101, Sigma-Aldrich, Switzerland) was used for the experiments. The fermentation temperature was 30 °C.

To measure the filter paper activity in the cultures, a modified IUPAC protocol was used. A I times 6 cm Whatman Nr.1 filter paper stripe is placed in a 2 mL Eppendorf vial and 1 mL 0.05 M citric acid buffer (pH 4.8) was added. Then, 0.5 mL enzyme solution (possibly diluted with citric acid buffer) was added and the mixture was incubated for 60 min at 50 °C in a water bath. After that the enzymes were deactivated by boiling the vials for 10 min in water. The solution was analyzed for glucose and cellobiose by HPLC (high pressure liquid chromatography). For concentrated enzyme preparations, the solutions had to be diluted so that during the essay slightly more and slightly less than 2 mg of glucose equivalents were released. For fermentation assays with low filter paper acitivity, 0.5 mL fermentation supernatant was added without dilution. For undiluted enzyme solutions, the filter paper activity expressed in FPU (filter paper unit)/mL could be calculated by multiplying the amount of glucose equivalents released with 0.185.

### Example 1

The membrane reactor was filled with 320 mL Mandels medium containing 20 g/L Avicel, inoculated with spores of *T*. *reesei* wild type, *T. reesei* Rut C30 and *Penicillium brasilianum* and incubated for 5 days at 30 °C. Then, the fermentation slurry was separated from the membrane without disrupting the biofilm which has developed on the membrane. The following hydrolysis was performed in shake flasks. Avicel at a concentration of 10 g/L and citric acid buffer (final concentration 0.05 M) was added to the following different hydrolysis mixtures:
- Supernatant:: The fermentation slurry has been centrifuged to remove all cells and remaining Avicel
- Whole cells.: The complete fermentation slurry was used
- Whole cells blank:: The complete fermentation slurry was used, but no fresh Avicel was added
- Membrane:: The biofilm covered membrane was added to the hydrolysis mixture.

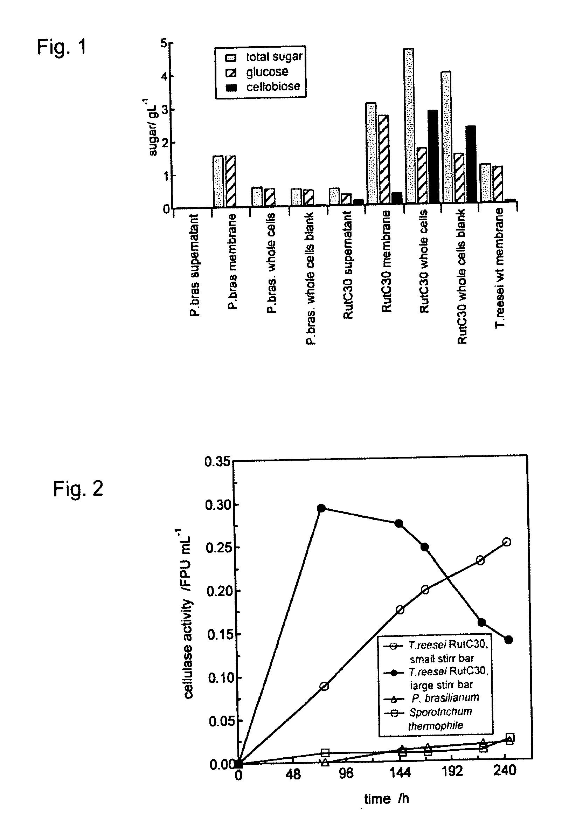

The total mass of the hydrolysis mixtures was 25 g. Flasks were incubated at 50 °C and with 150 rpm in an orbital shaker. Sugar concentrations were measured by HPLC as shown in Figure 1.

With the FPU assay in cell free supernatants a cellulase activity of 0.043 FPU/mL could be measured for the RutC30 fermentations. No activity could be measured in the other cultivations.

### Example 2

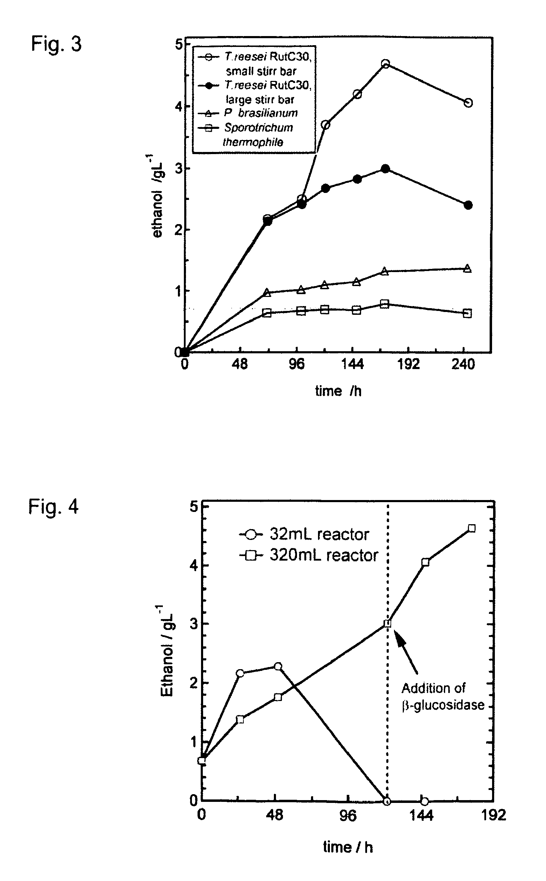

In the first step, the membrane reactor was filled with Mandels medium and 7.5 g/L Avicel and inoculated with *T. reesei* RutC30, P. brasilianum and Sporotrichum thermophile. In the RutC30 experiment, two different magnetic stirrer bars were tested, a large and a small one. The reactors were incubated for 10 days at 30 °C. Then, Avicel (10 g/L) and corn steep liquor (3 g/L) were added and the reactors were inoculated with S. *cerevisiae* cells. Figure 2 shows the cellulose activity prior to the inoculation with yeast cells, while Figure 3 shows the ethanol production after inoculation with yeast cells. Furthermore, beta glucosidase was added to the RutC30 experiments, as cellobiose accumulated which inhibits the cellulase. The results are shown in Figures 2 and 3.

### Example 3

In order to show the influence of the membrane area / liquid volume ratio on cellulase production and fermentation rate, the liquid volume of one reactor was decreased by a factor of 10 while the membrane area was kept constant. The reactors were filled with Mandels medium containing 7.5 g/L Avicel and inoculated with *T. reesei* RutC30 spores. After incubation for 189 h at 30 °C, yeast cells (to a final OD₆₀₀ of 0.5), Avicel (to a final concentration of 10 g/L) and corn steep liquor (to a final concentration of 3 g/L were added and the reactors were further incubated at 30 °C. The ethanol concentration was measured by HPLC, which is shown in Figure 4.

### Example 4

Dense Silicone membranes are permeable for water, ethanol, oxygen, CO₂ etc. by a solution diffusion process. Since the diffusion coefficients in silicone vary for the different substances, it is of interest to be able to estimate the mass transfer, i.e., how much oxygen enters the reactor and how much water and ethanol leave it. The mass transfer is usually characterized by an overall mass transfer coefficient kₒᵥ [m/h] following the equation *m* = *kₒᵥ ·* Δ*c* [g/(m²·h)].

The overall mass transfer coefficient for water through the dense silicone membrane was measured at 30 °C in an incubator using a gravimetrical approach. In this experiment the co-diffusion of a 2 %w/w (mass percentage solutions) ethanol solution in water was analyzed. Using the total membrane area of 12.6 cm², the mass flow rate of water *ṁ_{water}* is in the range of 3.4 · 10⁻³ g/cm² · h. Assuming the concentration of water in the bulk gas phase is zero, the concentration difference Δc is 1 g/cm³ and with that the overall mass transfer coefficient in the range of 3.4 · 10⁻⁵ g/h.

The overall mass transfer coefficient for ethanol through the dense silicone membrane was measured at 30 °C in an incubator by measuring the time course of the ethanol concentration in the liquid phase. Five different ethanol concentrations were used ranging from 2 to 10 g/L. The overall mass transfer coefficient was measured to be in the range of 4 to 7 · 10⁻⁴ g/h.

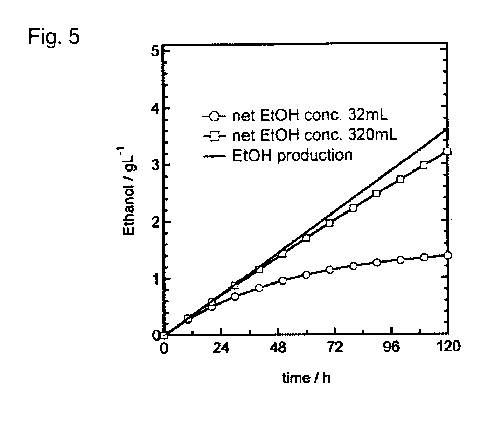

To illustrate the results of this phenomenon, a simple mathematical model was written. A constant ethanol production rate of 0.03 g/Lh was assumed and a constant overall mass transfer coefficient of 6.3 · 10⁻⁴ g/h. Reactor volumes of 30 and 300 mL were modeled. The resulting ethanol concentrations are shown in Figure 5.

Silicone membranes are slightly selective for ethanol and can therefore be used to enrich ethanol in aqueous solutions. This was shown in an experiment where the co-diffusion of a 2%w/w ethanol solution in water through the silicone membrane was analyzed. The mass flow rate of ethanol mₑₜₕₐₙₒₗ for an initial concentration of 20 g/L is 1.5 · 10⁻³ g/(cm²·h) while the mass flow rate of water m_{water} is in the range oaf 3.4 · 10⁻³ g/(cm²·h). This implies that using a silicone membrane the ratio between ethanol and water which leave the reactor is 0.3, i.e., the ethanol concentration in the (condensed) fluid, which diffused through the membrane is 300 g/L, and an ethanol enrichment by a factor of 15 could be achieved.

### DETAILED DESCRIPTION OF PREFERRED EMBODIMENTS

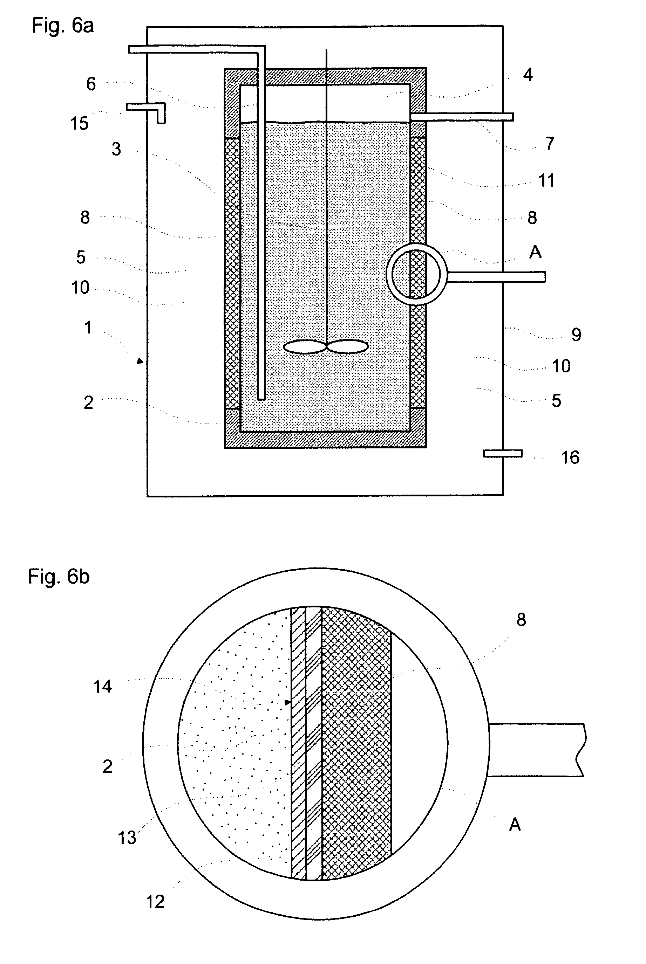

In Figure 6a a first embodiment of a stirred batch reactor 1 is shown. Dense polydimethylsiloxane membranes 8 (as e.g. sold by CM-CELFA Membrantechnik AG, Schwyz, Switzerland) represent a part of the vertical side wall of the reactor 1. The liquid reactor content 2 is mixed with the aid of a stirrer 3 and is covered with a small gaseous headspace 4. The membranes 8 separate the reactor 1 into an oxygen supply compartment 10 and into a fermentation compartment 11. The oxygen supply compartment 10 is sealed against the surrounding environment by a reactor wall 9. On their outside, i.e. with their surface facing the oxygen supply compartment 10, the membranes 8 are in contact with a medium, as e.g. a liquid or a gas 5, which contains oxygen (molecular oxygen). For the direct fermentative production of ethanol from lignocellulose using the reactor 1, fungi 12, e.g., *Trichoderma reesei, Aspergillus niger* or *Penicillium brasilianum,* producing lignocellulolytic enzymes, are combined with one or more ethanol producing microorganism(s) 13, e.g., *Saccharomyces cerevisiae, Zymomonas mobilis, Pichia stipitis, Escherichia coli* KO11, or *Klebsiella oxytoca.* The aerobic fungi 12 form part of a biofilm 14 located adjacent to the membrane 8, whereas the ethanol producing anaerobic microorganisms grow in the anaerobic parts of the reactor 1, namely in the parts of the biofilm 14 further away from the membrane 8 (layer 13) as well as in the liquid mixture 2, the suspension containing suspended lignocellulose and suspended anaerobic microorganisms (see Figure 6b). The whole pretreated biomass is used as substrate, including the liquid phase of the pretreatment step, which is further spiked with nutrients as e.g., corn steep liquor, phosphate, sulphate or vitamins. In a further development of the above described process, fresh substrate is continuously added through a short feed pipe 6 and excess reactor content is removed through nozzle 7, whereby a continuous process is achieved, which follows the physical laws of a continuous stirred tank reactor. The reactor 1 further comprises an inlet 15 and an outlet 16 for the supply and the removal of the oxygen containing gas or liquid 5 into resp. from the oxygen supply compartment 10.

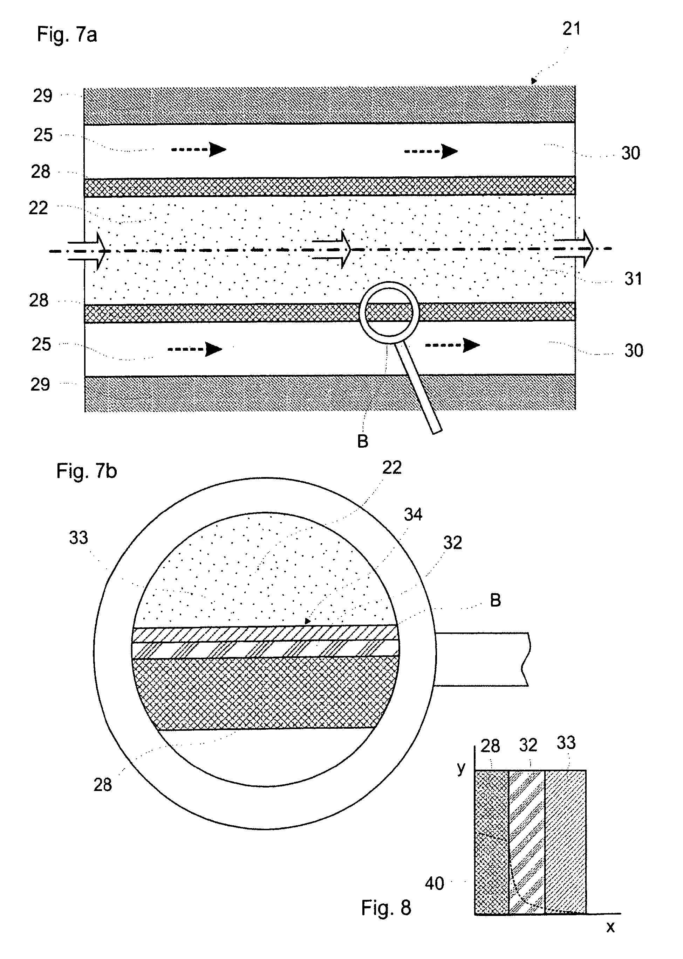

Figure 7a shows a second embodiment of the invention, which represents a plug flow reactor 21 for the continuous production of fermentation products from lignocellulose.

In an oxygen supply compartment 30 sealed against the surrounding environment by a reactor wall 29 and containing a medium, e.g. a liquid or a gas 25, which contains oxygen (molecular oxygen), are placed one or more tubular membranes 28, which consist e.g. of silicone tubing. A base material mixture 22 consisting of solid, pretreated biomass, the liquid phase of the pretreatment process and the necessary additional nutrient medium components, e.g. corn steep liquor, is axially passed through the fermentation compartment 31 of the reactor 21.

The process for the production of ethanol from lignocellulose in a biofilm reactor 21 according to the second embodiment runs basically as depicted in the description of Figure 6, with the main exemptions that the whole inner surface of the tubular membrane 28, which faces the fermentation compartment 31, is available as growth and aeration surface for the biofilm 34, and that the reactions follow the generally known advantageous characteristics of a plug flow reactor. I.e. the biofilm 34 contains aerobic fungi 32, which are located adjacent to the membrane 28, whereas the ethanol producing anaerobic microorganism 33 grow in the anaerobic parts of the reactor 21, namely in the parts of the biofilm 34 further away from the membrane 28 as well as in the nutrient medium 22, containing suspended lignocellulose and suspended anaerobic microorganisms (see also Figure 7b).

According to a further embodiment of a reactor the arrangement of the oxygen supply and fermentation compartments is vice versa in comparison with the reactor according to the second embodiment as shown in Fig. 7a, 7b. I.e., the oxygen supply compartment is located within the tube-like space, which is surrounded by the membrane and the fermentation compartment is located outside the tubular membrane encircling the circumferential surface of the membrane (not shown in the drawings).

According to a further development of the second embodiment the reactor contains one or more tubular membranes which are arranged e.g. in a winding manner within a reactor space. Also here, the oxygen supply compartment can be formed by the tubular space within the tubular membrane, whereas the fermentation compartment is formed by the reactor space housing the tubular membrane or a part of it and vice versa.

The membrane itself can be fixed on a drum-like support with perforations. The drum-like support may also be built from a mesh-like material. If the base material mixture has a high viscosity and therefore cannot be pumped into the reactor at the beginning of the process, it is also possible that the drum-like support on which the membrane is fixed or the whole reactor together with the tube-like membrane can be rotated. Furthermore, a screw conveyor can be provided, which conveys the highly viscous base material mixture within the reactor in flow direction at least at the beginning of the reactor until the base material mixture is fluidised.

Figure 8 shows schematically a possible distribution of oxygen 40 in the membrane 28 and in the biofilm 34 adjacent to the membrane 28. The y-axis expresses the oxygen concentration and the x-axis expresses the distance from the membrane surface.

The oxygen concentration considerably decreases within the layer of aerobic microorganism 32 on and near the surface of the membrane 28, so that within the layer of anaerobic microorganism 33 of the biofilm 34, adjoining the layer of aerobic microorganism 32 the oxygen content is depleted such that an anaerobic environment prevails.

While the invention has been described in present preferred embodiments of the invention, it is distinctly understood that the invention is not limited thereto, but may be otherwise variously embodied and practised within the scope of the claims.

## Claims

1. A method for the microbial production of fermentation products from organic feedstock in a reactor (1,21),
**characterized in that**
the reactor (1, 21) contains an oxygen permeable membrane (8, 28) separating an oxygen supply compartment (10, 30) from a fermentation compartment (11, 31), the oxygen permeable membrane (8, 28) contains a surface, which is facing the fermentation compartment (11, 31) and on which a biofilm (14, 34) is located, and the biofilm (14, 34) contains at least one strain of an aerobic microorganism (12, 32) in an aerobic zone of the fermentation compartment (11, 31) for the production of enzymes and the fermentation compartment (11, 31) further contains at least one strain of an anaerobic microorganism (13, 33) in an anaerobic zone of the fermentation compartment (11, 31) for the fermentation of fermentable sugars, wherein the at least one strain of aerobic microorganism is supplied with oxygen through the membrane (8, 28), and wherein in the fermentation compartment (11, 31) the following process steps take place:
a. production of enzymes for the enzymatic degradation of organic feedstock by the aerobic microorganism (12, 32) in the aerobic zone;
b. enzymatic degradation of the organic feedstock to fermentable sugars;
c. fermentation of the fermentable sugars to fermentation products by the anaerobic microorganism (13, 33) in the anaerobic zone.

2. A method according to claim 1, wherein the organic feedstock contains or consists of at least one of the following organic substances:
- polysaccharide, such as cellulose, hemicellulose or starch and
- lignocellulose
and wherein the biofilm (14, 34), dependent on the organic feedstock, contains at least one of the following strain of an aerobic microorganism:
- a strain of aerobic microorganisms, which produce cellulases for the decomposition of cellulose to fermentable sugars,
- a strain of aerobic microorganisms, which produce amylases for the decomposition of starch to fermentable sugars, and
- a strain of aerobic microorganisms, which produce lignin-modifying enzymes for the decomposition of lignin.

3. A method according to claim 1 or 2, wherein in the reactor (1, 21) the following steps take place:
d. removing of the fermentation product from the fermentation compartment, particularly by transporting the fermentation products through the membrane (8, 28) into the oxygen supply compartment (10, 30).

4. A method according to one of the claims 1 to 3, wherein in the reactor (1, 21) the oxygen permeable membrane (8, 28) separates an oxygen supply compartment (10, 30) and a fermentation compartment (11, 31), and oxygen is transported from the oxygen supply compartment (10, 30) to the fermentation compartment (11, 31) through the membrane and an oxygen zone is formed in the fermentation compartment (11, 31) adjacent to the membrane (8, 28) and the aerobic microorganism (12, 32) of the biofilm (14, 34) are located adjacent to the membrane (8, 28) in the aerobic zone and an oxygen gradient with decreasing oxygen content with increasing distance from the membrane (8, 28) is established within the biofilm (14, 34) located on the membrane (8, 28).

5. A method according to one of the claims 1 to 4, wherein the biofilm (14, 34) contains a consortium of at least two strains of microorganisms (12, 32; 13, 33), and wherein at least one of these strains of microorganisms (12, 32; 13, 33) is a strain of aerobic microorganism (12, 32) located adjacent to the surface of the membrane (8, 28) in the aerobic zone of the fermentation compartment (11, 31)) for the production of enzymes and wherein at least one of these strains of microorganisms (12, 32; 13, 33) for the fermentation of fermentable sugars is a strain of anaerobic microorganism (13, 33) located on the membrane (8, 28) in the biofilm (8, 28) in an oxygen depleted, preferably anaerobic zone and optionally also in the base material mixture (2, 22), which contains organic feedstock and which is supplied to the fermentation compartment (11,31), and the base material mixture (2, 22) is brought in contact with the biofilm (14, 34), wherein the aerobic microorganism (12, 32) in the biofilm (14, 34) produce enzymes for the enzymatic degradation of organic feedstock to fermentable sugar, and wherein the anaerobic microorganism (13, 33) in the biofilm (14, 34) and optionally also in the suspension (2, 22) ferment fermentable sugar into fermentation products.

6. A method according to one of the claims 1 to 5, wherein the supply of oxygen through the membrane (8, 28) is controlled, such that the oxygen content within the biofilm (14, 34) or within a zone in the base material mixture (2, 22) located next to the membrane (8, 28) and containing the biofilm (14, 34) decreases with increasing distance from the membrane (8, 28) to a level at which anaerobic condition are established, preferably decreases to approximately zero, so that the conditions in the base material mixture (2, 22) in the fermentation compartment (11, 31) beyond this zone are anaerobic.

7. A method according to one of the claims 1 to 6, wherein the fermentation products are removed from the fermentation compartment (11, 31) by transporting them through the membrane (8, 28) into the oxygen supply compartment (10, 30).

8. A method according to claim 7, wherein the fermentation products are removed from the oxygen supply compartment (10, 30) and separated from a gas or liquid phase, e.g. by means of condensation, adsorption or distillation.

9. A method according to one of the claims 1 to 8, wherein the oxygen and/or the fermentation products are transported through the membrane (8, 28) by means of a solution diffusion process.

10. A reactor (1, 21), particularly for carrying out the method according to one of the claims 1 to 9,
**characterized in that** the reactor (1,21) contains:
- an oxygen supply compartment (10, 30) for accommodating a fluid containing oxygen,
and
- a fermentation compartment (11, 31) for accommodating a base material mixture containing organic feedstock,
wherein the oxygen supply compartment (10, 30) and the fermentation compartment (11, 31) are separated by an oxygen permeable membrane (8, 28).

11. A reactor according to claim 10, wherein the reactor (1, 21) contains means for continuously or discontinuously supplying gaseous oxygen or oxygen dissolved in a liquid into the oxygen supply compartment (10, 30) and contains means for continuously or discontinuously supplying a base material mixture (2, 22) containing organic feedstock into the fermentation compartment (11, 31).

12. A reactor according to claim 10 or 11, wherein the surface of the membrane (8, 28) facing the fermentation compartment (11, 31) and therefore facing the base material mixture (2, 22) is covered with a biofilm (14, 34), which contains at least a strain of aerobic microorganism (12, 32) producing enzymes for the decomposition of organic material.

13. A reactor according to one of the claims 10 to 12, wherein the biofilm (14, 34) contains a consortium of at least two strains of microorganisms (12, 32; 13, 33), and wherein at least one of these strains of microorganisms is a strain of aerobic microorganism (12, 32) located adjacent to the surface of the membrane (8, 28) in an aerobic zone of the fermentation compartment (11, 31), and wherein at least one of these strains of microorganism is a strain of anaerobic microorganism (13, 33) located on the membrane (8, 28) in an oxygen depleted, preferably anaerobic, zone of the fermentation compartment (11, 31).

14. A reactor according to one of the claims 10 to 13, wherein the membrane is designed such that the fermentation products are removable from the fermentation compartment (11, 31) to the oxygen supply compartment (10, 30) by transportation through the membrane (8, 28) and means are provided for removing the fermentation products from the oxygen supply compartment (10, 30).

15. A reactor according to one of the claims 10 to 14, wherein the membrane (8, 28) is a tubular body and the oxygen supply compartment (10, 30) lies outside the tubular membrane (8, 28) and the fermentation compartment (11, 31) lies in the tube-like space within the tubular membrane (8, 28) or vice versa.
